# EUROPÄISCHE PATENTSCHRIFT

(11) **EP 2 221 079 B1**
(45) Veröffentlichungstag und Bekanntmachung des Hinweises auf die Patenterteilung: **14.09.2016**
(21) Anmeldenummer: 10001634.4
(22) Anmeldetag: 18.02.2010
(51) Int. Cl.: A61M 11/00, A61M 15/00, A61B 5/087, G01F 1/28

(54) **Austragvorrichtung zum Austrag einer pharmazeutischen Flüssigkeit in zerstäubter Form**
Dispensing device for dispensing a pharmaceutical liquid in atomised form
Dispositif de distribution d'un liquide pharmaceutique sous forme atomisée

(30) Priorität: 18.02.2009 DE 102009010565
(43) Veröffentlichungstag der Anmeldung: 25.08.2010
(73) Patentinhaber: Aptar Radolfzell GmbH, 78315 Radolfzell (DE)
(72) Erfinder: Körner, Joachim, 88690 Uhldingen-Mühlhofen (DE); Merk, Hans, 8560 Merstetten (CH); Umbeer, Volker, 78315 Radolfzell (DE); Zipfel, Steffen, 78315 Radolfzell (DE); Kohnle, Jörg, 78056 VS-Schwenningen (DE); Helmlinger, Michael, 78315 Radolfzell (DE); Keppner, Frank, 72458 Albstadt (DE)
(74) Vertreter: Patentanwaltskanzlei Cartagena

(56) Entgegenhaltungen:
- EP-A1- 0 461 281
- EP-A1- 0 933 092
- WO-A1-94/19042
- WO-A1-03/059423
- WO-A1-03/063937
- WO-A2-99/63946
- GB-A- 2 266 466
- US-A- 6 104 970
- US-A1- 2004 187 864
- US-A1- 2005 081 639
- US-A1- 2005 155 602

## Beschreibung

### Anwendungsgebiet und Stand der Technik

Die Erfindung betrifft eine Austragvorrichtung zum Austrag einer pharmazeutischen Flüssigkeit in zerstäubter Form. Diese gattungsgemäße Austragvorrichtung umfasst ein Gehäuse, ein Luftführungssystem mit mindestens einem Lufteinlass, mit einer Saugöffnung und mit mindestens einem Kanal, der den mindestens einen Lufteinlass mit der Saugöffnung verbindet, sowie einen Zerstäuber, der dazu ausgebildet ist, in Reaktion auf ein elektrisches Signal Flüssigkeit zu zerstäuben und der durch den Kanal strömenden Luft in zerstäubter Form zuzuführen. Dabei ist ein Sensorsystem zur Erkennung eines an der Saugöffnung erfolgenden Saugvorganges vorgesehen, welches einen Sensorflächenabschnitt aufweist, der in Reaktion auf den Saugvorgang durch eine durch den Saugvorgang verursachte Luftströmung verformt wird, wobei ein Sensor zur Erfassung dieser Verformung des Sensorflächenabschnitts vorgesehen ist.

Aus dem Stand der Technik sind derartige und ähnliche Austragvorrichtungen bekannt. Diese weisen eine Saugöffnung auf, die dafür vorgesehen ist, an den Mund oder die Nase eines Patienten angesetzt zu werden, so dass ein Gemisch aus Luft und zerstäubter Flüssigkeit im Zuge eines Einatmens aufgenommen werden kann. Diese Technik kann genutzt werden, um die Flüssigkeit in Form kleiner Tröpfchen beispielsweise bis tief in die Lunge zu fördern. Bei besonders einfachen Austragvorrichtungen dieses Typs muss der Patient das Einatmen und den Zerstäubungsvorgang selbstständig koordinieren, indem er während des Einatmens das Zerstäuben der pharmazeutischen Flüssigkeit beispielsweise mittels eines Betätigungstasters auslöst.

Diese Technik ist nachteilig, da Patienten zu einer einwandfreien Koordinierung des Einatmens und des Auslösens der Zerstäubung häufig nicht in der Lage sind. Dies gilt insbesondere für besonders junge und besonders alte Patienten.

Aus dem Stand der Technik sind daher auch die gattungsgemäßen Austragvorrichtungen bekannt. Bei diesen ist ein Sensorsystem vorgesehen, welches es gestattet, das Einatmen des Patienten zu erfassen und in Reaktion darauf automatisch den Zerstäuber zu aktivieren, so dass gleichsam das Einatmen selbst die Zerstäubung verursacht. Eine solche Austragvorrichtung ist beispielsweise aus der WO 2003/059423 A1 sowie der US 6,104,970 bekannt. US 2005 155602 beschreibt ein Sensorensystem im Hauptkanal eines Zerstäubers.

Aus der WO 2003/059423 A1 ist dabei bekannt, in den Kanal, durch den hindurch die Luft vom Patienten eingesogen wird, einen flexiblen Streifen einzubringen, der beim Einatmen des Patienten durch den Luftstrom ausgelenkt wird. Diese Auslenkung wird durch eine Polymerschicht auf dem Streifen erfassbar, da diese Polymerschicht bei der Verformung des Streifens ihren elektrischen Widerstand ändert. Durch eine Messung des elektrischen Widerstandes kann somit die Auslenkung beurteilt werden.

Als nachteilig an dieser aus der WO 2003/059423 A1 bekannten Lösung wird angesehen, dass der Streifen, der in etwa radial in den Strömungsweg der Luft hineinragt, ein signifikantes Hindernis für die Luft darstellt und den Strömungswiderstand deutlich erhöht. Der Patient muss daher besonders stark einatmen, um diese Strömungswiderstand zu überwinden und dennoch eine ausreichende Luftmenge einatmen zu können. Gerade bei krankheitsbedingt eingeschränkten Patienten kann dies schwierig sein. Der weiteren kann die exponierte Lage des Streifens dazu führen, dass sich Luftfeuchtigkeit auf dem Streifen niederschlägt. Dies kann negativen Einfluss auf die Messergebnisse haben.

### Aufgabe und Lösung

Aufgabe der Erfindung ist es, eine gattungsgemäße Austragvorrichtung dahingehend weiterzubilden, dass das Einatmen des Patienten sicher erkannt werden kann, ohne dass dies mit einer signifikanten Vergrößerung des Strömungswiderstandes einhergeht.

Erfindungsgemäß wird diese Aufgabe dadurch gelöst, dass das Luftführungssystem einen Hauptkanal aufweist, durch den ein Hauptanteil von mindestens 50% der bei einem Saugvorgang eingesaugten Luft entlang eines Strömungspfades entlang strömt, wobei der Sensorflächenabschnitt derart angeordnet ist, dass eine Strömungsrichtung des genannten Hauptanteils der Luft am Sensorflächenabschnitt parallel zur Ebene des Sensorflächenabschnitts verläuft oder mit der Ebene des Sensorflächenabschnitts einen Winkel von weniger als 20° einschließt.

Eine erfindungsgemäße Austragvorrichtung ist vorzugsweise als transportable Austragvorrichtung ausgebildet, die neben den genannten Komponenten gattungsgemäßer Austragvorrichtungen auch einen Medienspeicher und einen Energiespeicher umfasst. Das Gehäuse einer erfindungsgemäßen Austragvorrichtung weist als gasoffene Verbindungen nach außen primär die Saugöffnung auf, die vorzugsweise an eine Körperöffnung, nämlich den Mund oder die Nase, des Patienten hinsichtlich ihrer Form angepasst ist. Diese Saugöffnung dient als Austragsöffnung für das von der Austragvorrichtung erzeugte Luft-Tröpfchen-Gemisch. Die von dem Patienten durch die Saugöffnung eingeatmete Luft ist zuvor durch den mindestens einen Lufteinlass in das Gehäuse gelangt, wobei dieser Lufteinlass mit der Saugöffnung durch mindestens einen Kanal verbunden ist. Der Zerstäuber, der beispielsweise als elektrischer Piezozerstäuber ausgebildet sein kann, ist im Bereich dieses mindestens einen Kanals angeordnet. Die vom Zerstäuber in Form kleiner Tröpfen abgegebener Flüssigkeit vermengt sich dadurch mit der Luft in dem Kanal, so dass das erzeugte Aerosol durch die Saugöffnung ausgetragen werden kann.

Bei der beschriebenen erfindungsgemäßen Gestaltung ist das Sensorsystem der Austragvorrichtung im Bereich eines Hauptkanals angeordnet, wobei ein Hauptkanal im Sinne dieser Erfindung ein Kanal ist, durch den hindurch beim Anlegen eines Unterdrucks an der Saugöffnung mindestens 50% der an der Saugöffnung austretenden Luft transportiert wurde. Vorzugsweise ist dieser Anteil noch höher und liegt beispielsweise bei mindestens 66%, vorzugsweise bei mindestens 80%. Als Kanal im Zusammenhang mit dieser Erfindung wird der Bewegungspfad der Luft verstanden, ohne dass erheblich ist, ob der Kanal durch ein primär hierfür vorgesehenes beispielsweise rohrförmiges Bauteil oder aber durch Wandungen des Gehäuses der Austragvorrichtung und/oder andere Bestandteile der Austragvorrichtung begrenzt wird.

Der Sensorflächenabschnitt ist im Bereich des Hauptkanals angeordnet, erstreckt sich jedoch nicht quer zur Strömungsrichtung der Luft, sondern in etwa parallel zur Strömungsrichtung der Luft. Zumindest jedoch sollte der Sensorflächenabschnitt mit der Strömungsrichtung der Luft im Bereich des Sensorflächenabschnitts einen Winkel von weniger als 20° einschließen.

Diese Ausrichtung des Sensorflächenabschnitts in Richtung der Strömungsrichtung führt zu einem sehr geringen Strömungswiderstand. Das Einatmen durch den Patienten wird daher durch den Sensorflächenabschnitt nicht wesentlich verschlechtert.

Je nach Anordnung des Sensorflächenabschnitts am oder im Hauptkanal sind unterschiedliche Arten des Auslenkung des Sensorflächenabschnitts möglich. So kann es bei einem Saugvorgang an der Saugöffnung in Abhängigkeit der Ausgestaltung und der Anordnung des Sensorflächenabschnitts zu einem Schwingen oder aber zu einer statischen Auslenkung des Sensorflächenabschnitts kommen. Unabhängig von der Art der Auslenkung kann diese durch den Sensor erfasst werden und an ein Steuergerät der Austragvorrichtung zur Weiterverarbeitung übermittelt werden. Der Grad der statischen oder dynamischen Verformung des Sensorflächenabschnitts lässt Rückschlüsse darauf zu, wie kräftig der Patient einatmet.

Als besonders vorteilhaft wird es angesehen, wenn der Sensorflächenabschnitt an einer Innenwandung des Hauptkanals vorgesehen ist. Bei einer solchen Gestaltung bildet der Sensorflächenabschnitt gleichsam selbst einen Teil der Wandung und verringert somit den verursachten Strömungswiderstand nochmals.

Vorzugsweise überdeckt der Sensorflächenabschnitt dabei zumindest überwiegend eine Durchbrechung in der Innenwandung des Hauptkanals. Dies führt dazu, dass beim Durchströmen der Luft durch den Hauptkanal der hierdurch erzeugte Unterdruck den Sensorflächenabschnitt in den Hauptkanal hineinzieht, so dass dieser in gut messbarer Form ausgelenkt bzw. verformt wird. Um diese Wirkung noch zu verstärken, kann der Sensorflächenabschnitt im Bereich einer Blende oder Einschnürung des Hauptkanals angeordnet sein, da aufgrund der höheren Strömungsgeschwindigkeit in einem solchen Bereich ein stärkerer Druckabfall die Folge ist, wodurch der Sensorflächenabschnitt in verstärktem Maße in den Hauptkanal hineingezogen wird.

Die Erfindung betrifft gemeinsam mit der oben beschriebenen Gestaltung eine gattungsgemäße Austragvorrichtung, bei der das Luftführungssystem einen Nebenkanal aufweist, durch den ein Nebenanteil von maximal 20% der bei einem Saugvorgang angesaugten Luft entlang eines Strömungspfades entlang strömt, wobei der Nebenanteil vorzugsweise noch geringer ist und insbesondere vorzugsweise bei maximal 15% der angesaugten Luft liegt. Dabei ist der Sensorflächenabschnitt in diesem Nebenkanal vorgesehen, vorzugsweise im Bereich eines Zusammenflusses, an dem der Nebenkanal in den Hauptkanal mündet.

Diese Gestaltung einer erfindungsgemäßen Austragvorrichtung sieht vor, dass die Erfassung des Einatmens durch den Patienten einen erheblichen Anteil der eingesaugten Luft hinsichtlich des zu überwindenden Strömungswiderstandes nicht tangiert. Mindestens 80% der Luft werden bei dieser Gestaltung durch einen oder mehrere Kanäle vom jeweiligen Lufteinlass zur Ansaugöffnung gefördert, die durch den Sensorflächenabschnitt nicht beeinflusst werden. Der Sensorflächenabschnitt ist stattdessen in einem Nebenkanal vorgesehen, durch den hindurch beim Anlegen eines Unterdrucks an der Saugöffnung maximal 20%, vorzugsweise maximal 15%, insbesondere maximal 10%, der geförderten Luft transportiert werden. Ungeachtet der Gestaltung und der Ausrichtung des Sensorflächenabschnitts ist bei dieser Ausführungsform somit durch den Sensorflächenabschnitt eine Erhöhung des Strömungswiderstandes um maximal etwa 20% zu erwarten.

Vorzugsweise ist der Sensorflächenabschnitt an einem Übergangsbereich zwischen dem Nebenkanal und dem Hauptkanal vorgesehen. Er erstreckt sich vorzugsweise derart, dass er den Querschnitt des Nebenkanals zumindest überwiegend überdeckt und/oder den Querschnitt des Hauptkanals nicht oder nur in geringem Maße beeinflusst. Als überwiegende Überdeckung des Nebenkanals wird es angesehen, wenn der Sensorflächenabschnitt in der Projektion entlang der Erstreckungsrichtung des Nebenkanals zumindest 50% der Querschnittsfläche des Nebenkanals verdeckt. Vorzugsweise verdeckt der Sensorflächenabschnitt jedoch einen höheren Anteil von 80% oder 90%.

Hinsichtlich der Gestaltung des Sensorflächenabschnitts sind insbesondere zwei Weiterbildungen vorteilhaft.

Bei der ersten dieser Weiterbildungen ist vorgesehen, dass der Sensorflächenabschnitt zumindest an einer Einspannkante gehäusefest festgelegt ist und an einer der Einspannkante gegenüberliegenden Kante quer zur Erstreckungsebene des Sensorflächenabschnitts frei auslenkbar ist. Diese Gestaltung ist von Vorteil, da sie bei einfachem Aufbau eine vergleichsweise starke und damit gut erfassbare Auslenkung des Sensorflächenabschnitts gestattet. Der Sensorflächenabschnitt ist bei einer solchen Gestaltung vorzugsweise nur entlang einer geraden Kante befestigt, während die übrigen Kanten auslenkbar sind. Vorzugsweise ist der Sensorflächenabschnitt dieser Gestaltung auf eine durch ihn abgedeckte Öffnung, wie beispielsweise die Einmündung eines Nebenkanals, derart abgestimmt, dass er nur im Bereich schmaler Spalten zwischen der Öffnung und den freien Kanten umströmt werden kann, wobei die Summe der Flächen dieser Spalte vorzugsweise weniger als 20% der abgedeckten Öffnung beträgt, so dass der Luftstrom durch die abgedeckte Öffnung eine besonders starke Auslenkung des Sensorflächenabschnitts zur Folge hat.

Eine zweite Variante sieht vor, dass der Sensorflächenabschnitt an einer Durchbrechung des Hauptkanals vorgesehen ist, wobei die Durchbrechung durch den Sensorflächenabschnitt gasdicht verschlossen wird. Bei einer solchen Gestaltung ist der Sensorflächenabschnitt somit umlaufend in beständigem Kontakt zu einer Wandung des Hauptkanals. Die Verformung des Sensorflächenabschnitts erfolgt demnach durch eine elastische Dehnung der Fläche des Sensorflächenabschnitts. Der Vorteil bei dieser Gestaltung ist, dass keine zusätzliche Luft in den Hauptkanal einströmt, da durch die umlaufende Verbindung in einfacher Art und Weise die Gasdichtheit hergestellt werden kann.

Der Sensor zur Erfassung der Verformung des Sensorflächenabschnitts muss nicht zwingend am Sensorflächenabschnitt selbst vorgesehen sein. Es ist beispielsweise auch möglich, durch eine Lichtschranke oder eine ähnliche Erkennungssensorik die Auslenkung des Sensorflächenabschnitts zu erfassen. Als vorteilhaft wird jedoch eine Ausgestaltung angesehen, bei der der Sensorflächenabschnitt eine Trägerfläche aufweist, auf der der Sensor angebracht ist, wobei dieser dazu ausgebildet ist, die Verformung der Trägerfläche zu erfassen. Diese Gestaltung erlaubt es somit, an dem Sensorflächenabschnitt selbst die Verformung zu erfassen, ohne hierfür eine vom Sensorflächenabschnitt beabstandete Sensorik zu benötigen. Als Sensor kommen dabei beispielsweise Dehnungsmessstreifen oder andere auslenkungsabhängig variierende elektrischen Widerstände in Frage.

Als besonders vorteilhaft wird es jedoch angesehen, wenn der Sensor als Piezosensor ausgebildet ist, wobei dieser Piezosensor vorzugsweise als Piezolack oder Piezofolie auf dem Sensorflächenabschnitt aufgebracht ist.

Ein solcher Piezosensor zeichnet sich dadurch aus, dass durch ihn bereits sehr geringe Verformungen erfassbar sind. Bei Verformung oder Auslenkung des Sensorflächenabschnitts erzeugt der Piezosensor eine Spannung, die Rückschlüsse auf den Grad der Verformung bzw. Auslenkung zulässt. Der Piezosensor ist insbesondere aufgrund der Anordnung des Sensorflächenabschnitts von Vorteil, da die erfindungsgemäße Anordnung mit einer vergleichsweise geringen Auslenkung des Sensorflächenabschnitts einhergeht, so dass es erforderlich ist, auch diese vergleichsweise geringe Auslenkung zuverlässig zu erkennen und auswerten zu können. Der besondere Vorteil eines Piezosensors liegt auch darin, dass dieser einen einfachen Aufbau mit einer unmittelbar auswertbaren Ausgangsspannung verbindet. Dies ist insbesondere gegenüber Sensoren ein Vorteil, die verformungsabhängig ihren elektrischen Widerstand ändern, da bei solchen Sensoren eine zusätzliche Brückenschaltung zur Auswertung erforderlich ist.

Der Piezosensor kann so ausgebildet sein, dass eine Längenveränderung des Sensors in Erstreckungsrichtung des Sensorflächenabschnitts zu einer auswertbaren Piezospannung führt. Eine solche Längenveränderung, insbesondere eine Dehnung, findet beim Auslenken des Sensorflächenabschnitts statt.

Alternativ kann der Piezosensor auch so ausgebildet sein, dass eine Stauchung des Sensorflächenabschnitts orthogonal zu dessen Erstreckungsrichtung zu einer auswertbaren Piezospannung führt. Eine solche Stauchung orthogonal zur Erstreckungsrichtung erfolgt zwangsläufig bei der Auslenkung des Sensorflächenabschnitts. Besonders von Vorteil ist es, wenn der Piezosensor in Form eines Piezolacks auf dem Sensorflächenabschnitt bzw. dessen Trägerfläche angebracht ist. Alternativ hierzu ist es auch möglich, eine Piezofolie zu verwenden, die auf dem Sensorflächenabschnitt bzw. dessen Trägerfläche angebracht wird.

Bei einer Weiterbildung der Erfindung weist die Austragvorrichtung ein Steuergerät zur Auswertung des Sensors auf, wobei das Ausgangssignal des Sensors vor der Auswertung durch das Steuergerät vorzugsweise durch einen Tiefpassfilter gefiltert wird und/oder durch einen Verstärker verstärkt wird.

Das Steuergerät, welches vorzugsweise innerhalb des Gehäuses der Austragvorrichtung vorgesehen ist, kann das Ausgangssignal des Sensor in Hinblick darauf auswerten, ob eine Auslenkung stattgefunden hat, die als Atmen des Patienten identifiziert wird. Insbesondere bei der Verwendung eines Piezosensors wird es als vorteilhaft angesehen, wenn die Ausgangsspannung des Piezosensors durch einen Verstärker verstärkt wird, um die anschließende Auswertung zu erleichtern. Ebenfalls als vorteilhaft wird es angesehen, einen Tiefpassfilter vorzusehen, der hochfrequente Auslenkungen/Verformungen des Sensorflächenabschnitts herausfiltert, da solche hochfrequenten Änderungen zumeist das Ergebnis von Erschütterungen sind. Vorzugsweise werden Frequenzen oberhalb von 5 Hz, vorzugsweise oberhalb von 2 Hz, insbesondere vorzugsweise oberhalb von 1 Hz, durch den Tiefpassfilter aus dem Ausgangssignal des Sensors entfernt.

Bei einer Weiterbildung der Erfindung ist das Steuergerät dafür ausgebildet, den Zerstäuber zu aktivieren, sobald die durch den Sensor erfasste Verformung des Sensorflächenabschnitts einen vorgegebenen Schwellwert erreicht oder überschritten hat. Dieser Schwellwert ist dabei auf einen Wert festgelegt, dessen Überschreitung durch das Ausgangssignal des Sensors bedeutet, dass es sich erstens um eine starke Auslenkung handelt, die nicht auf einer Erschütterung der Austragvorrichtung zurückzuführen ist, und dass es sich zweitens um eine Auslenkung im Zuge eines Einatmens handelt, die Ergebnis eines ausreichend starken Einatmens ist. Das Steuergerät kann zusätzlich dafür ausgebildet sein, Einatmungsvorgänge durch den Patienten zu erkennen, die nicht ausreichend stark für einen bestimmungsgemäßen Austragsvorgang sind, und dem Patienten dies beispielsweise durch ein akustisches Signal mitzuteilen.

### Kurzbeschreibung der Zeichnungen

Weitere Aspekte und Vorteile der Erfindung ergeben sich außer aus den Ansprüchen auch der nachfolgenden Beschreibung von bevorzugten Ausführungsbeispielen der Erfindung, welche nachfolgend anhand der Darstellungen verdeutlicht werden. Dabei zeigen:
- Fig. 1: eine erste Ausführungsform einer erfindungsgemäßen Austragvorrichtung in einer schematischen geschnittenen Seitenansicht,
- Fig. 2: einen Hauptkanal der Austragvorrichtung gemäß Fig. 1 in einer perspektivischen Ansicht,
- Fig. 3a und 3b: die Austragvorrichtung der Fig. 1 während der Benutzung und
- Fig. 4: eine zweite Ausführungsform einer erfindungsgemäßen Austragsvorrichtung in einer schematischen geschnittenen Seitenansicht.

### Detaillierte Beschreibung der Ausführungsbeispiele

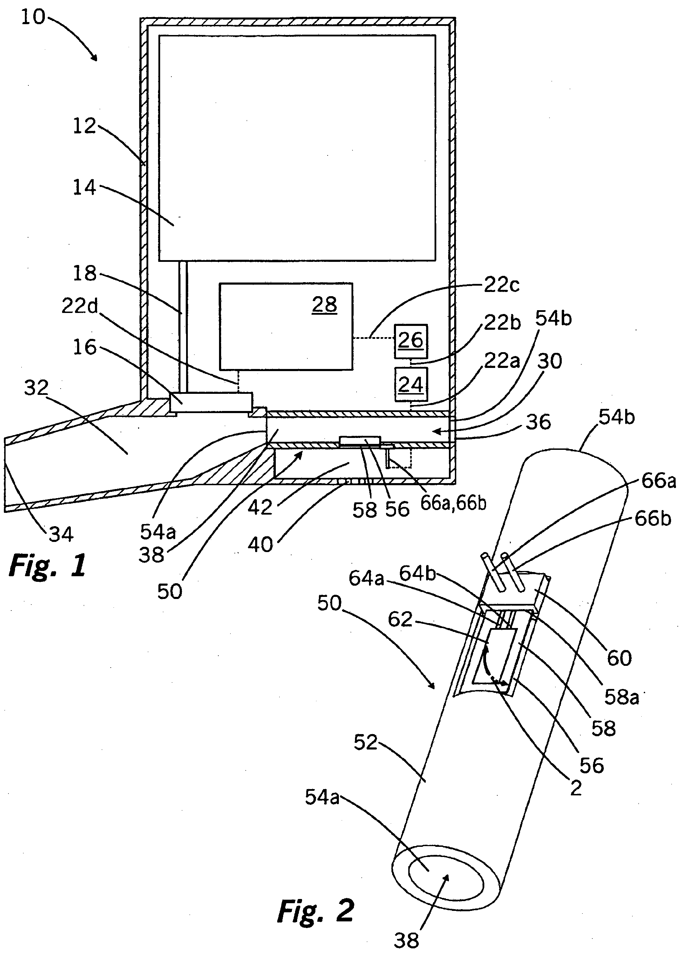

Fig. 1 zeigt eine erste Austragvorrichtung 10 gemäß der Erfindung. Diese Austragvorrichtung 10 weist ein Gehäuse 12 auf, innerhalb dessen ein Flüssigkeitsspeicher 14, ein Piezozerstäuber 16 und ein den Flüssigkeitsspeicher 14 und den Piezozerstäuber 16 verbindender Flüssigkeitskanal 18 vorgesehen sind. Weiterhin ist innerhalb des Gehäuses 12 ein Luftführungssystem 30 vorgesehen. Dieses Luftführungssystem 30 weist einen Auslassbereich 32 mit einer Saugöffnung 34 auf. In diesem Auslassbereich 32 ist auch der bereits genannte Piezozerstäuber 16 vorgesehen. Zur Versorgung des Luftführungssystems 30 mit Luft ist zum einen ein Haupteinlass 36 vorgesehen, der über einen Hauptkanal 38 mit dem Auslassbereich 32 verbunden ist. Darüber hinaus sind Einlassschlitze 40 an der Unterseite des Geräts vorgesehen, die über einen als Nebenkanal wirkenden Freiraum 42 durch eine Öffnung 56 mit dem Hauptkanal 38 verbunden sind. Diese Öffnung 56 ist im dargestellten unbenutzten Zustand durch einen Sensorflächenabschnitt 58 zumindest größtenteils verschlossen.

Fig. 2 zeigt eine Hauptkanalbaugruppe 50. Diese Hauptkanalbaugruppe 50 befindet sich im eingebauten Zustand in der Austragvorrichtung 10 der Fig. 1. Die Hauptkanalbaugruppe 50 weist den bereits beschriebenen Hauptkanal 38 auf, der von einem rohrförmigen Abschnitt 52 umgeben ist, wobei der rohrförmige Abschnitt 52 an seinen Stirnenden 54a, 54b offen ausgebildet ist. Wie der Fig. 1 zu entnehmen ist, verbinden diese beiden offenen Stirnenden 54a, 54b im eingebauten Zustand den Hauptkanal 38 einerseits mit dem Auslassbereich 32 und andererseits mit dem Haupteinlass 36. In der Wandung des Rohrabschnittes 52 ist die bereits beschriebene Durchbrechung 56 vorgesehen. Diese ist durch den ebenfalls bereits beschriebenen Sensorflächenabschnitt 58 weitgehend verschlossen. Der Sensorflächenabschnitt 58 ist etwa rechteckig geformt und an einer kurzen Seite 58a mit einem Trägerabschnitt 60 verbunden, der seinerseits an der Wandung des Rohrabschnitts 52 festgelegt ist.

Auf dem Sensorflächenabschnitt ist eine Piezofolie 62 angebracht, die bei einer Auslenkung des Sensorflächenabschnitts 58 in Richtung des Pfeils 2 zu einer Piezospannung an den beiden Folienkontakten 64a, 64b führt. Zur besseren Abgreifbarkeit dieser Spannung sind zwei Anschlusspins 66a, 66b vorgesehen, die in nicht näher dargestellter Art und Weise mit jeweils einem der Folienkontakte 64a, 64b verbunden sind.

Wie aus Fig. 1 hervorgeht, sind diese beiden Pins 66a, 66b über Leitungen 22a mit einem Tiefpassfilter 24 verbunden. Dieser ist dafür ausgebildet, aus dem Ausgangssignal des Sensors 62 Frequenzen oberhalb von 5 Hz herauszufiltern. Der Tiefpassfilter 24 ist über weitere Leitungen 22b mit einem Verstärker 26 verbunden, der die tiefpassgefilterte Spannung des Ausgangssignals des Piezosensors 62 verstärkt. Über weitere Leitungen 22c ist der Verstärker 26 mit dem Steuergerät 28 verbunden.

Dieses Steuergerät 28 erhält somit das tiefpassgefilterte und verstärkte Ausgangssignal des Piezosensors 62. In Abhängigkeit dieses Ausgangssignals kann das Steuergerät 28 über eine Leitung 22d den Zerstäuber 16 aktivieren, so dass dieser die über den Flüssigkeitskanal 18 zugeführte Flüssigkeit in Form feiner Tröpfchen in den Auslassbereich 32 abgibt.

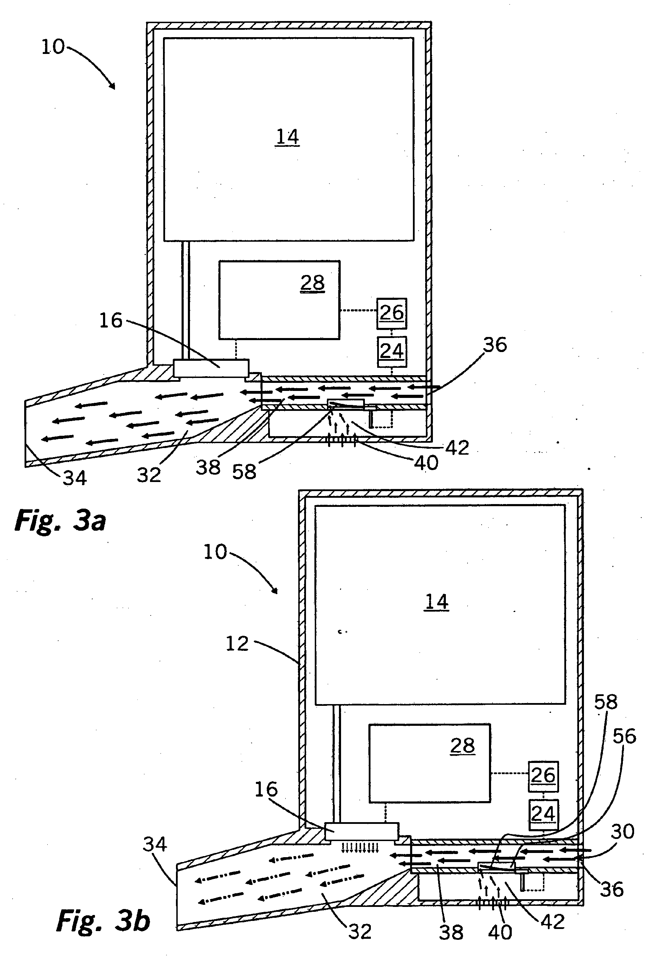

Die dargestellte und beschriebene Gestaltung dient der automatischen Aktivierung des Flüssigkeitszerstäubers 16, sobald ein Patient ausreichend stark an der Saugöffnung 34 saugt. Dies ist anhand der Fig. 3a und 3b verdeutlicht.

Sobald der Patient an der Saugöffnung 34 saugt, kommt es innerhalb des Luftführungssystems 30 zu Luftbewegungen. Dies ist in Fig. 3a darstellt. Zu einem überwiegenden Teil von über 90% wird die Luft, die an der Saugöffnung 34 abgegeben und vom Patienten eingeatmet wird, durch den Haupteinlass 36 in das Gehäuse 12 gesogen und durch den Hauptkanal 38 bis in den Auslassbereich 32 gefördert. Von dort aus kann die Luft weiter bis zur Saugöffnung 34 gelangen. Der Saugvorgang führt innerhalb des Hauptkanals 38 zu einem Unterdruck, durch den der Sensorflächenabschnitt 58 in den Hauptkanal 38 hinein ausgelenkt wird. Dabei gelangt in geringem Maße auch ein Anteil von weniger als 10% der Luft durch die Durchbrechung 56 in den Hauptkanal 38, die nicht durch den Haupteinlass 36 in das Gehäuse eingeströmt ist, sondern durch die Einlassschlitze 40. Die Einlassschlitze 40 brauchen hierfür nicht groß zu sein. Sie müssen lediglich so groß sein, dass sich in dem Nebenkanal 42 kein Unterdruck bildet, der der Auslenkung des Sensorflächenabschnitts 58 entgegensteht.

Die Auslenkung des Sensorflächenabschnitts 58 kann in gefilterter und verstärkter Form vom Steuergerät 28 erfasst werden. Wenn diese Auslenkung ausreichend groß ist und/oder ausreichend lang andauert, kann das Steuergerät 28 darauf schließen, dass es sich nicht nur um eine kurze Erschütterung der Austragvorrichtung 10 oder ein nur schwaches Einatmen durch den Patienten handelt. In Reaktion auf dieses Auswertungsergebnis aktiviert das Steuergerät 28 den Flüssigkeitszerstäuber 16.

Fig. 3b zeigt, wie die Aktivierung des Flüssigkeitszerstäubers 16 dazu führt, dass Flüssigkeit in fein zerstäubter Form in den Auslassbereich 32 der Austragvorrichtung 10 gelangt und dort von dem Luftstrom mitgerissen wird. Hieraus ergibt sich das gewünschte Aerosol, welches der Patient durch die Saugöffnung 34 hindurch einatmet.

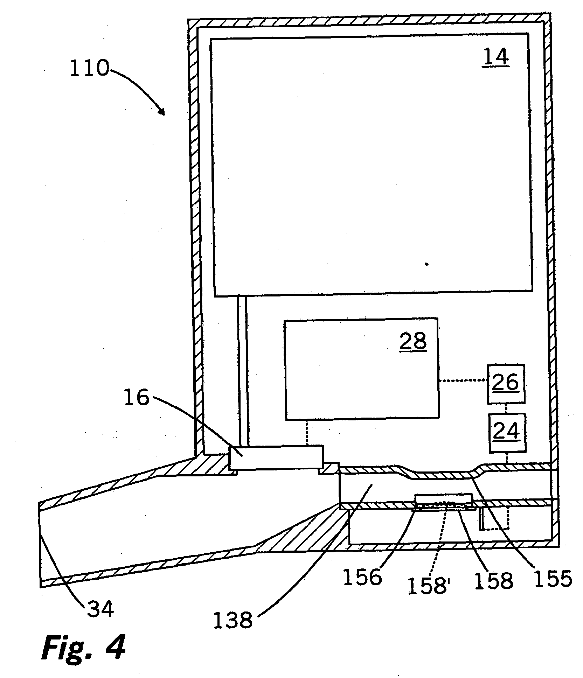

Eine alternative Ausführungsform einer erfindungsgemäßen Austragvorrichtung ist in Fig. 4 dargestellt. Diese Austragvorrichtung 110 der Fig. 4 stimmt mit der Austragvorrichtung der Fig. 1 bis 3 weitgehend überein. Für identische oder weitgehend identische Komponenten werden daher die gleichen Bezugsziffern verwendet.

Der wesentliche Unterschied zwischen der Ausführungsform der vorangegangenen Figuren 1 bis 3 und der Ausführungsform der Fig. 4 liegt darin, dass der Sensorflächenabschnitt 158 im Falle der Ausführungsform der Fig. 4 die Durchbrechung 156 vollständig gasdicht verschließt. Da somit an dieser Stelle keine Luft in den Hauptkanal 138 einströmen kann, bedarf es auch keiner Einlasschlitze wie bei der Ausführungsform der Fig. 1 bis 3. Sobald der Patient an der Saugöffnung 34 einatmet, wird bei der Gestaltung der Fig. 4 ein Unterdruck im Hauptkanal 138 erzeugt, wobei dieser Unterdruck dazu führt, dass der membranartige Sensorflächenabschnitt 158 in den Hauptkanal 138 hineingezogen wird. Dieser Effekt wird noch dadurch verstärkt, dass die Durchbrechung 156 im Bereich einer Einschnürung bzw. Blende 155 vorgesehen ist. Die Verformung des Sensorflächenabschnitts 158 ist in Fig. 4 anhand der gestrichelten Linien 158' verdeutlicht. Die Auswertung der Verformung des Sensorflächenabschnitts 158 erfolgt jedoch auch bei dieser Ausgestaltung der Fig. 4 in der bereits erläuterten Art und Weise über einen Piezofoliensensor auf dem Sensorflächenabschnitt 158, der im Zuge der Verformung des Sensorflächenabschnitts 158 gedehnt wird und dadurch eine für das Steuergerät 28 erfassbare Spannung verursacht.

Die dargestellten Ausführungsformen bieten durch die Anordnung und Ausgestaltung der Sensorflächenabschnitte eine vorteilhafte Erfassbarkeit des Einatmungsvorgangs, ohne dabei den Strömungswiderstand des Luftführungssystems deutlich zu verschlechtern. Aufgrund der nur geringen Auslenkung der Sensorflächenabschnitte ist die Verwendung von Piezosensoren besonders vorteilhaft, da diese bereits eine geringe Verformung bzw. Auslenkung der Sensorflächenabschnitte zuverlässig erfassen können.

## Patentansprüche

1. Austragvorrichtung (10) zum Austrag einer pharmazeutischen Flüssigkeit in zerstäubter Form, umfassend
- ein Gehäuse (12),
- ein Luftführungssystem (30) mit
- mindestens einem Lufteinlass (36, 40),
- einer Saugöffnung (34) und
- mindestens einem Kanal (32, 38, 42), der den mindestens einen Lufteinlass mit der Saugöffnung verbindet, und
- einen Zerstäuber (16), der dafür ausgebildet ist, in Reaktion auf ein elektrisches Signal Flüssigkeit zu zerstäuben und der durch den Kanal (32) strömenden Luft in zerstäubter Form zuzuführen,
wobei
- ein Sensorsystem (58, 62, 24, 26) zur Erkennung eines an der Saugöffnung (34) erfolgenden Saugvorgangs vorgesehen ist, welches einen Sensorflächenabschnitt (58) aufweist, der in Reaktion auf den Saugvorgang durch eine durch den Saugvorgang verursachte Luftströmung verformt wird, wobei ein Sensor (62) zur Erfassung dieser Verformung des Sensorflächenabschnitts (58) vorgesehen ist,
**dadurch gekennzeichnet, dass**
das Luftführungssystem (30) einen Hauptkanal (38) sowie einen Nebenkanal (42) aufweist, wobei durch den Hauptkanal (38) ein Hauptanteil von mindestens 50% und durch den Nebenkanal (42) ein Nebenanteil von maximal 20% der bei einem Saugvorgang angesaugten Luft entlang eines Strömungspfades entlang strömt und wobei der Sensorflächenabschnitt (58) in dem Nebenkanal (42) vorgesehen ist und derart angeordnet ist, dass eine Strömungsrichtung des Hauptanteils am Sensorflächenabschnitt (58) parallel zur Ebene der Sensorflächenabschnitts verläuft oder mit der Ebene des Sensorflächenabschnitts einen Winkel von weniger als 20° einschließt.

2. Austragvorrichtung nach Anspruch 1,
**dadurch gekennzeichnet, dass**
der Sensorflächenabschnitt (58) an einer Innenwandung (52) des Hauptkanals (138) vorgesehen ist.

3. Austragvorrichtung nach einem der vorstehenden Ansprüche,
**dadurch gekennzeichnet, dass**
der Sensorflächenabschnitt (58) in diesem Nebenkanal (42) im Bereich eines Zusammenflusses, an dem der Nebenkanal (42) in den Hauptkanal (38) mündet, vorgesehen ist.

4. Austragvorrichtung nach einem der vorstehenden Ansprüche,
**dadurch gekennzeichnet, dass**
der Sensorflächenabschnitt (58) einen Querschnitt des Nebenkanals (42) zumindest überwiegend überdeckt.

5. Austragvorrichtung nach einem der vorstehenden Ansprüche,
**dadurch gekennzeichnet, dass**
der Sensorflächenabschnitt (58) zumindest an einer Einspannkante (58a) gehäusefest festgelegt ist und an einer der Einspannkante gegenüberliegenden Kante quer zur Erstreckungsebene des Sensorflächenabschnitts (58) frei auslenkbar ist.

6. Austragvorrichtung nach einem der vorstehenden Ansprüche,
**dadurch gekennzeichnet, dass**
der Sensorflächenabschnitt (58) eine Trägerfläche aufweist, auf der der Sensor (62) angebracht ist, der dafür ausgebildet ist, die Verformung der Trägerfläche zu erfassen.

7. Austragvorrichtung nach einem der vorstehenden Ansprüche,
**dadurch gekennzeichnet, dass**
der Sensor (62) als Piezosensor (62) ausgebildet ist, wobei dieser Piezosensor vorzugsweise als Piezolack oder Piezofolie (62) auf dem Sensorflächenabschnitt (58) flächig aufgebracht ist.

8. Austragvorrichtung nach Anspruch 7,
**dadurch gekennzeichnet, dass**
ein Steuergerät (28) zur Auswertung des Sensors (62) vorgesehen ist, wobei das Ausgangssignal des Sensors vor der Auswertung vorzugsweise durch einen Tiefpassfilter (24 gefiltert wird und/oder durch einen Verstärker (26) verstärkt wird.

9. Austragvorrichtung nach Anspruch 8,
**dadurch gekennzeichnet, dass**
das Steuergerät (28) dafür ausgebildet ist, den Zerstäuber (16) zu aktivieren, sobald die durch den Sensor (62) erfasst Verformung des Sensorflächenabschnitts (58) einen vorgegebenen Schwellwert überschritten hat.

## Claims

1. Dispensing device (10) for dispensing a pharmaceutical liquid in atomized form, comprising
- a housing (12),
- an air-guiding system (30) with
- at least one air inlet (36, 40),
- a suction opening (34) and
- at least one channel (32, 38, 42), which connects the at least one air inlet to the suction opening, and
- an atomizer (16), which is designed to atomize liquid in reaction to an electrical signal and to deliver the air flowing through the channel (32) in atomized form,
wherein
- a sensor system (58, 62, 24, 26) is provided for detecting a suction process occurring at the suction opening (34), said sensor system having a sensor surface portion (58) which, in reaction to the suction process, is deformed by an air stream caused by the suction process, wherein a sensor (62) is provided for detecting this deformation of the sensor surface portion (58),
**characterized in that**
the air-guiding system (30) has a main channel (38) and a secondary channel (42), wherein a main part of at least 50% of the suctioned air flows along a flow path through the main channel (38) and a secondary part of at most 20% of the suctioned air flows along a flow path through the secondary channel (42), and wherein the sensor surface portion (58) is provided in the secondary channel (42) and is arranged in such a way that a direction of flow of the main part on the sensor surface portion (58) runs parallel to the plane of the sensor surface portion or encloses an angle of less than 20° with the plane of the sensor surface portion.

2. Dispensing device according to Claim 1, **characterized in that** the sensor surface portion (58) is provided on an inner wall (52) of the main channel (138).

3. Dispensing device according to either of the preceding claims, **characterized in that** the sensor surface portion (58) in this secondary channel (42) is provided in the area of a confluence where the secondary channel (42) opens into the main channel (38).

4. Dispensing device according to one of the preceding claims, **characterized in that** the sensor surface portion (58) covers at least most of a cross section of the secondary channel (42).

5. Dispensing device according to one of the preceding claims, **characterized in that** the sensor surface portion (58) is secured to the housing at least on a clamping edge (58a) and, on an edge lying opposite the clamping edge, is freely movable transversely with respect to the plane of extent of the sensor surface portion (58).

6. Dispensing device according to one of the preceding claims, **characterized in that** the sensor surface portion (58) has a carrier surface on which the sensor (62) is mounted which is designed to detect the deformation of the carrier surface.

7. Dispensing device according to one of the preceding claims, **characterized in that** the sensor (62) is designed as a piezoelectric sensor (62), wherein this piezoelectric sensor is preferably mounted in two-dimensional form as a piezoelectric paint or piezoelectric film (62) on the sensor surface portion (58).

8. Dispensing device according to Claim 7, **characterized in that** a controller (28) is provided for evaluating the sensor (62), wherein the output signal of the sensor, prior to the evaluation, is filtered preferably by a low-pass filter (24) and/or amplified by an amplifier (26).

9. Dispensing device according to Claim 8, **characterized in that** the controller (28) is designed to activate the atomizer (16) as soon as the deformation of the sensor suface portion (58) detected by the sensor (62) has exceeded a predefined threshold value.

## Revendications

1. Dispositif de distribution de liquide (10) destiné à délivrer un liquide pharmaceutique sous forme atomisée, comprenant
- un boîtier (12),
- un système d'amenée d'air (30) ayant
- au moins une entrée d'air (36, 40),
- une ouverture d'aspiration (34) et
- au moins un canal (32, 38, 42) qui relie l'au moins une entrée d'air à l'ouverture d'aspiration, et
- un atomiseur (16) qui est conçu pour atomiser un liquide en réaction à un signal électrique et pour l'introduire sous forme atomisée dans l'air s'écoulant par l'intermédiaire du canal (32),
dans lequel
- il est prévu un système de capteur (58, 62, 24, 26) destiné à détecter un processus d'aspiration se produisant au niveau de l'ouverture d'aspiration (34), comportant une partie de surface de capteur (58) qui est déformée en réaction au processus d'aspiration par une circulation d'air provoquée par le processus d'aspiration, dans lequel il est prévu un capteur (62) destiné à détecter ladite déformation de la partie de surface de capteur (58),
**caractérisé en ce que** le système d'amenée d'air (30) comporte un canal principal (38) ainsi qu'un canal secondaire (42), dans lequel une proportion majoritaire d'au moins 50% de l'air aspiré lors d'un processus d'aspiration circule par l'intermédiaire du canal principal (38) et une proportion secondaire d'au plus 20% de l'air aspiré lors du processus d'aspiration circule par l'intermédiaire du canal secondaire (42) le long d'un trajet de circulation et dans lequel la partie de surface de capteur (58) est prévue dans le canal secondaire (42) et est disposée de telle manière qu'un sens de circulation de la proportion majoritaire s'étende sur la partie de surface de capteur (58) parallèlement au plan de la partie de surface ou forme avec le plan de la partie de surface un angle inférieur à 20°.

2. Dispositif de distribution de liquide selon la revendication 1,
**caractérisé en ce que** la partie de surface de capteur (58) est prévue sur une paroi interne (52) du canal principal (138).

3. Dispositif de distribution de liquide selon l'une quelconque des revendications précédentes,
**caractérisé en ce que** la partie de surface de capteur (58) est prévue dans ledit canal secondaire (42) dans la région d'une confluence au niveau de laquelle le canal secondaire (42) débouche dans le canal principal (38).

4. Dispositif de distribution de liquide selon l'une quelconque des revendications précédentes,
**caractérisé en ce que** la partie de surface de capteur (58) recouvre au moins majoritairement une section transversale du canal secondaire (42).

5. Dispositif de distribution de liquide selon l'une quelconque des revendications précédentes,
**caractérisé en ce que** la partie de surface (58) est fixée solidaire du boîtier au moins sur un bord de serrage (58a) et peut être librement déviée sur un bord opposé au bord de serrage transversalement au plan d'extension de la partie de surface de capteur (58).

6. Dispositif de distribution de liquide selon l'une quelconque des revendications précédentes,
**caractérisé en ce que** la partie de surface de capteur (58) comporte une surface de support sur laquelle est monté le capteur (62), qui est conçue pour détecter la déformation de la surface de support.

7. Dispositif de distribution de liquide selon l'une quelconque des revendications précédentes,
**caractérisé en ce que** le capteur (62) est réalisé sous la forme d'un capteur piézoélectrique (62), dans lequel ledit capteur piézoélectrique est de préférence appliqué à plat sur la partie de surface de capteur (58) sous la forme d'une peinture piézoélectrique ou d'une feuille piézoélectrique (62).

8. Dispositif de distribution de liquide selon la revendication 7,
**caractérisé en ce qu'**il est prévu un appareil de commande (28) destiné à évaluer le capteur (62), dans lequel le signal de sortie du capteur est de préférence filtré par un filtre passe-bas (24) et/ou amplifié par un amplificateur (26) avant l'évaluation.

9. Dispositif de distribution de liquide selon la revendication 8,
**caractérisé en ce que** l'appareil de commande (28) est conçu pour activer l'atomiseur (16) dès que la déformation de la partie de surface de capteur (58) détectée par le capteur (62) a dépassé une valeur de seuil prédéterminée.
